Europäisches Patentamt

European Patent Office

Office européen des brevets

(19)

(11) Veröffentlichungsnummer : **0 140 063**
**B1**

(12) # EUROPÄISCHE PATENTSCHRIFT

(45) Veröffentlichungstag der Patentschrift :
02.03.88

(21) Anmeldenummer : 84110669.3

(22) Anmeldetag : 07.09.84

(51) Int. Cl.⁴ : **A 61 F   2/16**

(54) Multifokale, insbesondere bifokale intraokulare künstliche Augenlinse.

(30) Priorität : 07.09.83 DE 3332313

(43) Veröffentlichungstag der Anmeldung :
08.05.85 Patentblatt 85/19

(45) Bekanntmachung des Hinweises auf die Patenterteilung : 02.03.88 Patentblatt 88/09

(84) Benannte Vertragsstaaten :
AT DE FR GB IT NL

(56) Entgegenhaltungen :
US-A- 4 435 856

(73) Patentinhaber : Achatz, Manfred, Dr.
Feldstrasse 11
D-6477 Limeshain 2 (DE)

Höfer, Peter
Karlsbader Strasse 50
D-8750 Aschaffenburg (DE)

Strobel, Jürgen, Dr.
Zeppelinstrasse 14a
D-3550 Marburg (DE)

(72) Erfinder : Achatz, Manfred, Dr.
Feldstrasse 11
D-6477 Limeshain 2 (DE)
Erfinder : Höfer, Peter
Karlsbader Strasse 50
D-8750 Aschaffenburg (DE)
Erfinder : Strobel, Jürgen, Dr.
Zeppelinstrasse 14a
D-3550 Marburg (DE)

(74) Vertreter : Nöth, Heinz, Dipl.-Phys. Patentanwalt
Mozartstrasse 17
D-8000 München 2 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine multifokale, insbesondere bifokale, intraokulare künstliche Augenlinse mit einem die Pupille der Iris abdeckenden optischen Linsenteil aus durchsichtigem Material.

Eine künstliche bifokale Augenlinse nach dem alternierenden bzw. Wechselprinzip, bei der entweder nur der Nahbereich oder nur der Fernbereich der Sehhilfe im Strahlengang liegt und damit wirksam ist, ist aus der US-A-40 10 496 bekannt. Diese Linse ist im unteren Linsenteil mit einem segmentförmigen Nahteil versehen. Der segmentförmige Nahteil und der darüber liegende segmentförmige Fernteil treffen in einer Trennungslinie aufeinander. Ungünstig ist bei diesem Linsentyp, daß an der Trennungslinie ein Bildsprung auftritt. Diese Bildsprünge resultieren daraus, daß das zentrale Nervensystem zu träge ist, innerhalb kürzester Zeit verschiedene, auf unterschiedlichen Netzhautplätzen entstehende Bildeindrücke zu verschmelzen. Ferner hat es sich gezeigt, daß dann, wenn nicht wenigstens 3/4 des Pupillenbereichs von der jeweiligen Schärfezone abgedeckt ist, sich deutlich Doppelbilder und Kontrastminderungen zeigen. Es ist daher äußerst schwierig, die richtige Segmenthöhe bzw. den richtigen Verlauf der Trennlinie festzulegen.

Aufgabe der Erfindung ist es daher, eine künstliche Augenlinse der eingangs genannten Art zu schaffen, mit der auf der Netzhaut gleichzeitig Bilder von Gegenständen in unterschiedlicher Entfernung vom Betrachter erzeugt werden, so daß durch das zentrale Nervensystem dasjenige Bild, auf das sich der Träger der künstlichen intraokularen Augenlinse konzentriert, ohne Wechsel des Bildeindruckes auf den Netzhautplätzen als scharfes Bild ausgewählt wird.

Diese Aufgabe wird durch die im Patentanspruch 1 angegebenen Merkmale gelöst.

In vorteilhafter Weise wird durch die Erfindung eine Intraokularlinse nach dem simultanen Prinzip geschaffen, bei der nach der Implantation ein scharfes Sehen sowohl im Nah- als auch im Fernbereich ohne zusätzliche Sehhilfen möglich ist, wobei sowohl die Linsenteile für das Nahsehen als auch die Linsenteile für das Fernsehen gleichzeitig im Strahlengang liegen. Durch Feststellen des Pupillendurchmessers entweder während der Operation oder später durch medikamentöse bzw. mikrochirurgische Maßnahmen lassen sich die optischen Linsenteile einwandfrei in den Strahlengang bringen.

Die intraokulare künstliche Augenlinse kann unterschiedlich ausgelegt sein, z. B. als Hinterkammer-, Vorderkammer- oder irisfixierte Linse.

Die Intraokularlinse nach dem simultanen Prinzip erhält man durch die konzentrische Anordnung von Nah- und Fernbereich, durch vertikale Aufteilung der Linsenfläche in eine Zone mit Nahwirkung und eine Zone mit Fernwirkung sowie durch die Aufteilung der Linsenfläche in radial sich erstreckende Bereiche mit Nah- und Fernwirkung.

Bei der Ausführungsform, bei der die optisch wirksame Fläche der Intraokularlinse in die Nah- und Fernbereichszonen in eine innere Kreisfläche und mehrere konzentrische Kreisringflächen aufgeteilt ist, die in radialer Richtung alternierend angeordnet sind, wird noch erreicht, daß bei raschem Hell-Dunkelwechsel das Sehvermögen nicht beeinträchtigt wird. Bei der Intraokularlinse wird das Flächenverhältnis der Flächen der kreis- und kreisringförmigen Nah- und Fernteile, ausgehend von der Linsenmitte in radialer Richtung zum Linsenrand hin, jeweils konstant gehalten wird. Wenn sich bei raschem Hell-Dunkelwechsel die Pupille rasch öffnet, bleibt das Flächenverhältnis der Nah- und Fernbereichszonen gleich, wodurch Visusminderung vermieden und Beeinträchtigungen beim Sehen verhindert sind.

Wenn im Zentrum des optischen Linsenteils der Fernteil angeordnet ist und außen der Nahteil, kann die optische Wirkung der konzentrischen Kreisringflächen, welche den Nahteil und den Fernteil bilden, auch radial nach außen progressiv verlaufen. Das heißt dann, daß der Scheitelbrechwert von innen nach außen zunimmt, wobei diese Scheitelbrechwertzunahme radial von innen nach außen bevorzugt kontinuierlich erfolgt. Wenn im umgekehrten Fall der Nahteil im Zentrum des optischen Linsenteils und der Fernteil außen angeordnet sind, kann die optische Wirkung der konzentrischen Kreisringflächen, welche den Nahteil und den Fernteil bilden, progressiv radial nach innen verlaufen. Das heißt dann, daß der Scheitelbrechwert von innen nach außen abnimmt, wobei diese Scheitelbrechwertabnahme radial von innen nach außen bevorzugt kontinuierlich erfolgt.

Auch ist es möglich, die Nah- und Fernbereichzonen in mehrere Sektoren mit gleichen Winkeln aufzuteilen und diese alternierend um die optische Achse anzuordnen.

Ferner ist es möglich, die Nah- und Fernbereichzone je in einer Hälfte des optischen Linsenteils vorzusehen, wobei die Trennlinie zwischen der Nahbereichzone und der Fernbereichzone bei in das Auge implantierter Linse vom oberen Linsenrand zum unteren Linsenrand verläuft und die Nahbereichzone im nasalen (näher zur Nase des Trägers liegenden) und die Fernbereichzone im temporalen (entfernt zur Nase des Trägers liegenden) Linsenbezirk liegen. Auch hierbei wirken sich Leuchtdichteunterschiede nicht aus, und die Linse ist unabhängig vom Pupillenspiel. Auch bei einer infolge schwacher Beleuchtung und bei Nacht auftretenden Pupillenerweiterung führt dies nicht zu einem verstärkten Verschwommensehen, weil die Anteile, mit denen der Fernteil und der Nahteil im Pupillenbereich gleichzeitig abgedeckt werden, gleich bleiben.

Bei den Linsen nach der Erfindung werden Bilder von Gegenständen in der Ferne und der Nähe auf der Netzhaut gleichzeitig abgebildet. Im zentralen Nervensystem wird dasjenige Bild ausgewählt, auf das sich der Träger der künstlichen intraokularen Augenlinse konzentriert. Ein Bildsprung wie bei der bekannten alternierenden bifokalen Augenlinse tritt nicht auf. Die Nah- und Fernbereichzonen können auf der Vorder- und/oder Rückfläche des optischen Linsenteils gebildet sein. Die optischen Wirkungen der Nah- und Fernbereichzonen können durch entsprechende Flächenbearbeitung des Linsenkörpers oder durch Kombination von Materialien unterschiedlicher Brechungsindizes erzielt werden. Zur Erzielung eines stenopäischen Effekts, d. h. einer größeren Tiefenschärfe wie beim Lochkameraeffekt, kann das Linsenmaterial peripher so abgeschirmt bzw. abgedunkelt sein, daß im Zentrum ein Sehloch verbleibt, z. B. mit einem Durchmesser in der Größenordnung von 0,5 bis 2 mm. Durch dieses Sehloch wird ein Objekt mittels eines engen Strahlenbündels angebildet. Dadurch werden die Zerstreuungskreise auf der Netzhaut des fehlsichtigen Auges kleiner und die Bildschärfe somit verbessert.

Anhand der beiliegenden Figuren wird an Ausführungsbeispielen die Erfindung noch näher erläutert. Es zeigt :

Fig. 1 eine erstes Ausführungsbeispiel für eine bivisuelle künstliche Intraokularlinse, bei dem eine Nah- und eine Fernbereichzone konzentrisch zueinander angeordnet sind ;

Fig. 2 ein Ausführungsbeispiel für eine künstliche, intraokulare Augenlinse, bei dem Nah- und Fernbereichzonen von einer Kreisfläche und konzentrischen Kreisringflächen gebildet sind ;

Fig. 3 ein Ausführungsbeispiel, bei dem der optische Linsenteil in zwei Hälften durch eine senkrechte Trennungslinie in eine Nah- und Fernbereichzone geteilt ist, und

Fig. 4 ein Ausführungsbeispiel mit sektorförmigen Nah- und Fernbereichzonen.

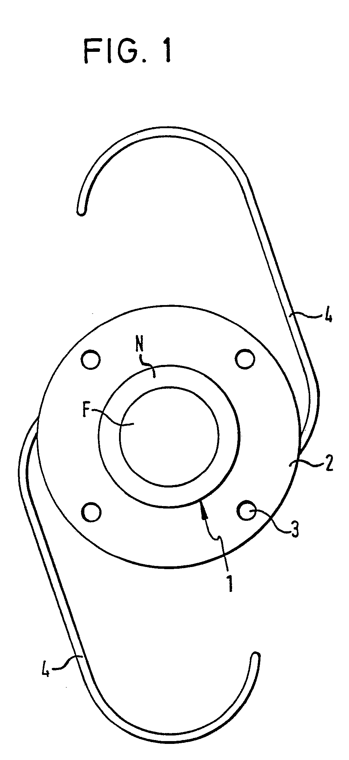

Beim Ausführungsbeispiel einer bivisuellen Intraokularlinse der Fig. 1 besitzt ein optischer Linsenteil 1 eine in der Mitte angeordnete Fernbereichzone F in Kreisflächenform und konzentrisch um diese in Kreisringflächenform eine Nahbereichzone N. Es kann jedoch auch der Fernteil F innen und der Nahteil N außen angeordnet sein. Der Linsenkörper besitzt Bohrungen 3 möglichst nahe am Umfangsrand der Linse in einem den optischen Linsenteil 1 umgebenden peripheren kreisringförmigen Linsenteil 2, damit die optische Funktionsweise der Linse nicht gestört wird. Haltebügel 4 dienen zur Befestigung der Linse im Auge.

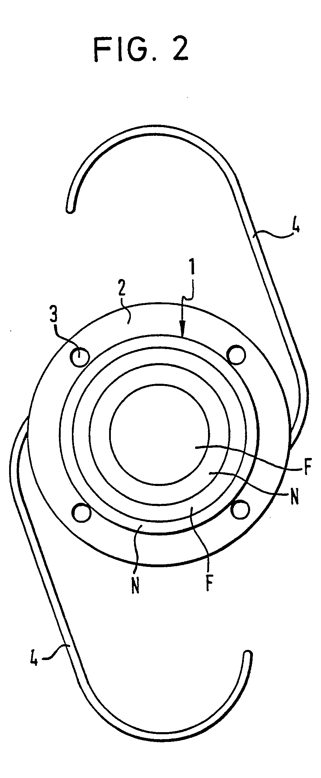

Das in der Fig. 2 dargestellte Ausführungsbeispiel einer multifokalen, intraokularen künstlichen Augenlinse besitzt in der Mitte des optischen Linsenteils 1 eine Fernbereichzone F in Kreisflächenform und eine konzentrisch um diese angeordnete kreisringförmige Nahbereichzone N, auf diese folgend radial nach außen eine kreisringförmige konzentrisch angeordnete Fernbereichzone F und eine kreisringförmige konzentrisch angeordnete Nahbereichzone N. Es ist jedoch auch möglich, im Zentrum des optischen Linsenteils 1 die Nahbereichzone N und um diese eine konzentrische kreisringförmige Fernbereichzone F usw. anzuordnen. Im peripheren kreisringförmigen Linsenteil 2, der optisch nicht wirksam ist, sind die Bohrungen 3 vorgesehen, mit denen, wie beim Ausführungsbeispiel der Fig. 1, gegebenenfalls nach der Implantation der Linse vor dem endgültigen Schließen des Augapfels die Linse in eine geeignete Position gedreht werden kann. Diese Bohrungen 3 sind so angeordnet, daß sie die optische Funktionsweise der Linse nicht beeinträchtigen. Die Linse besitzt ferner die Haltebügel 4, mit denen die Linse fixiert werden kann.

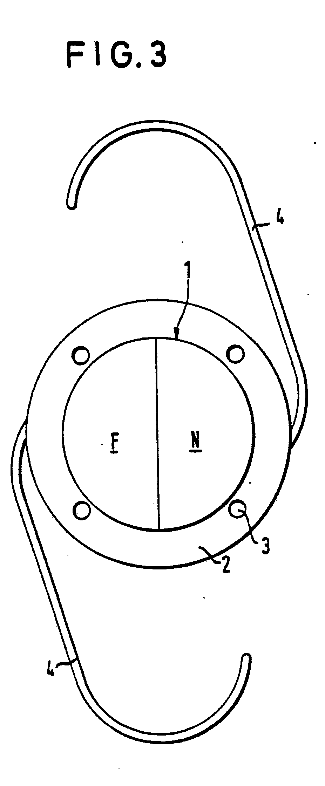

Das Ausführungsbeispiel der Fig. 3 ist vom bivisuellen Typ wie das Ausführungsbeispiel der Fig. 1, jedoch verläuft die Trennlinie zwischen der Nahbereichzone N und der Fernbereichzone F bei eingesetztem Linsenkörper vom oberen Linsenrand zum unteren Linsenrand und trennt den optischen Linsenteil 1 in zwei Hälften, von denen die eine Hälfte die Fernbereichzone F und die andere Hälfte die Nahbereichzone N bildet. Bei ins Auge eingesetzter Linse befindet sich die Nahbereichzone N näher zur Nase des Trägers als die Fernbereichzone F. Auch bei diesem Ausführungsbeispiel sind die Bohrungen 3 in einem Linsenbereich nahe dem Linsenrand angeordnet, so daß die optische Funktionsweise der Linse nicht beeinträchtigt wird. Haltebügel 4 dienen zum Festlegen der Linse im Auge.

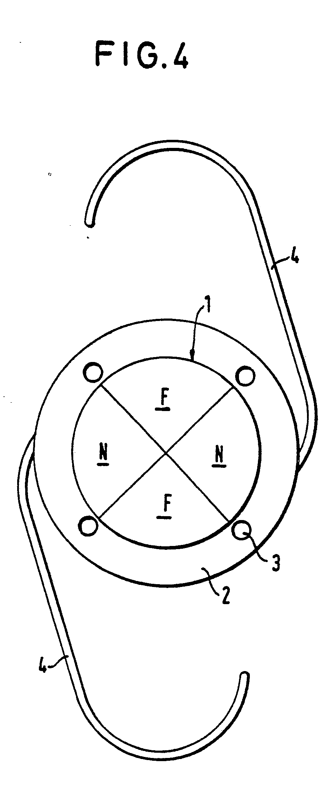

Beim Ausführungsbeispiel der Fig. 4 sind zwei Fernbereichzonen F und zwei Nahbereichzonen N in Sektorform mit gleichen Sektorwinkeln vorgesehen. Beim dargestellten Ausführungsbeispiel sind die Sektorwinkel 90°. Es ist jedoch auch möglich, mehr Nah- und Fernbereichzonen mit entsprechend geringeren Sektorwinkeln vorzusehen. Die Nah- und Fernbereichzonen N und F sind alternierend um die Linsenachse angeordnet. Bohrungen 3 befinden sich in einem peripheren Linsenteil 2, der optisch unwirksam ist. Befestigungsmittel 4 dienen ebenfalls zum Festlegen der Linse im Auge.

Für die künstliche Augenlinse können auch andere Befestigungsmittel vorgesehen sein. Bekannte Befestigungsmittel sind beschrieben in den deutschen Offenlegungsschriften 25 04 540, 26 05 847, 26 54 999 und 27 25 219.

**Patentansprüche**

1. Multifokale, insbesondere bifokale, intraokulare künstliche Augenlinse mit einem die Pupille der Iris abdeckenden optischen Linsenteil (1) aus durchsichtigem Material, dadurch gekennzeichnet, daß die Nahbereichs- und Fernbereichzone oder- zonen (N, F) jeweils etwa gleiche Flächenanteile aufweisen und auf dem optischen Linsenteil (1) entweder konzentrisch zueinander angeordnet sind oder durch diametrale Aufteilung der Linsenfläche in eine Zone mit Nahwirkung und eine Zone mit Fernwirkung gebildet sind oder durch Aufteilung der Linsenfläche in radial sich erstreckende Bereiche mit Nah- und Fernwirkung gebildet sind.

2. Augenlinse nach Anspruch 1, dadurch gekennzeichnet, daß Nah- und Fernbereiche konzentrisch zueinander angeordnet sind.

3. Augenlinse nach Anspruch 2, dadurch gekennzeichnet, daß die Nah- und Fernbereichszonen (N, F) in eine innere Kreisfläche und mehrere konzentrische Kreisringflächen aufgeteilt sind, die in radialer Richtung alternierend angeordnet sind.

4. Augenlinse nach einem der Ansprüche 2 und 3, dadurch gekennzeichnet, daß im Zentrum des optischen Linsenteils (1) der Fernteil (F) angeordnet ist.

5. Augenlinse nach Anspruch 4, dadurch gekennzeichnet, daß die optische Wirkung der konzentrischen Kreisringflächen radial nach außen progressiv verläuft.

6. Augenlinse nach einem der Ansprüche 2 und 3, dadurch gekennzeichnet, daß im Zentrum des optischen Linsenteils der Nahteil (N) und der Fernteil (F) außen angeordnet sind.

7. Augenlinse nach Anspruch 6, dadurch gekennzeichnet, daß die optische Wirkung der konzentrischen Kreisringflächen radial nach innen progressiv verläuft.

8. Augenlinse nach Anspruch 1, dadurch gekennzeichnet, daß die Nah- und Fernbereichzonen (N, F) in mehrere radial sich erstreckende Sektoren mit gleichen Winkeln aufgeteilt sind, die um die optische Achse alternierend angeordnet sind.

9. Augenlinse nach Anspruch 1, dadurch gekennzeichnet, daß die Nah- und Fernbereichzone (N, F) je in einer Hälfte des optischen Linsenteils (1) vorgesehen sind, wobei bei in das Auge implantierter Linse der Übergangsbereich zwischen Nah- und Fernteil vom oberen zum unteren Linsenrand verläuft und den optischen Linsenteil (1) in einen nasalen (näher zur Nase des Trägers liegenden) und einen temporalen (entfernt zur Nase des Trägers liegenden) Linsenbezirk aufteilt und daß die Nahbereichzone (N) im nasalen und die Fernbereichzone (F) im temporalen Linsenbezirk liegen.

10. Augenlinse nach einem der Ansprüche 1 bis 9, dadurch gekennzeichnet, daß die Nah- und Fernbereichzonen (N, F) auf der Vorder- und/oder Rückfläche des optischen Linsenteils (1) gebildet sind.

11. Augenlinse nach Anspruch 10, dadurch gekennzeichnet, daß zur Erzielung der Fernteil- und Nahteilwirkung auf der Vorder- und Rückfläche des optischen Linsenteils die Linse bikonvex gekrümmt ist.

12. Augenlinse nach einem der Ansprüche 1 bis 9, dadurch gekennzeichnet, daß die Nah- und Fernbereichzonen (N, F) durch Materialien unterschiedlicher Brechungsindizes gebildet sind.

13. Augenlinse nach einem der Ansprüche 1 bis 12, dadurch gekennzeichnet, daß die Nah- und Fernbereichzonen (N, F) durch ein Material mit in radialer Richtung aufweisenden Brechungsindex-Gradienten gebildet sind.

14. Augenlinse nach einem der Ansprüche 1 bis 13, dadurch gekennzeichnet, daß die Nah- und Fernbereichzonen (N, F) durch Flächenbearbeitung des optischen Linsenteils (1) gebildet sind.

15. Augenlinse nach Anspruch 1, dadurch gekennzeichnet, daß die optisch wirksame Fläche im Zentrum zur Erzielung eines stenopäischen Effekts begrenzt ist.

16. Augenlinse nach Anspruch 15, dadurch gekennzeichnet, daß im Zentrum des Linsenkörpers ein Sehloch gebildet ist, das von teilweise oder ganz lichtundurchlässigem Linsenmaterial umgrenzt ist.

**Claims**

1. A multifocal, particularly bifocal, intraocular artificial eye lens comprising an optical lens portion (1) which is made of transparent material and covers the pupil of the iris, characterized in that the near-range and farrange zone or zones (N, F) occupy approximately equal areas and are either mutually concentrically arranged on the optical lens portion or have been formed in that the lens area has been diametrically divided into a near-range zone and a far-range zone or in that the lens area has been divided into radially extending near-range and far-range zones.

2. An eye lens according to claim 1, characterized in that the near-range and far-range zones are concentric to each other.

3. An eye lens according to claim 2, characterized in that the near-range and far-range zone or zones (N, F) are divided into an inner circular area and a plurality of concentric annular circular areas, which alternate in a radial direction.

4. An eye lens according to claim 2 or 3, characterized in that the far-range zone (F) is disposed at the centre of the optical lens portion (1).

5. An eye lens according to claim 4, characterized in that the optical action of the concentric circular annular areas is progressive in an outward radial direction.

6. An eye lens according to claim 2 or 3, characterized in that the near-range zone (N) is disposed at the centre of the optical lens portion and the far-range zone (F) is disposed on the outside.

7. An eye lens according to claim 6, characterized in that the optical action of the concentric circular annular areas is progressive in an inward radial direction.

8. An eye lens according to claim 1, characterized in that the near-range and the far-range zones (N, F) are divided into a plurality of radially extending sectors including equal angels and alternating around the optical axis.

9. An eye lens according to claim 1, characterized in that the near-range and far-range zones (N, F) are provided in respective halves of the optical lens portion (1) and when the lens has been implanted into the eye the transitional region between the near-range and far-range zones extends from the top to the bottom rim portions of the lens and divides the optical lens portion into a nasal lens region (which is closer to the nose of the wearer) and a temporal lens region (which is remote from the nose of the wearer) and the near-range zone (N) is disposed in the nasal lens region and the far-range zone (F) is disposed in the temporal lens region.

10. An eye lens according to any of claims 1 to 9, characterized in that the near-range and far-range zones (N, F) are formed on the front and/or rear surface of the optical lens portion (1).

11. An eye lens according to claim 10, characterized in that the lens has a biconvex curvature to provide farrange and near-range actions on the front and rear surfaces of the optical lens portion.

12. An eye lens according to any of claims 1 to 9, characterized in that the near-range and far-range zones (N, F) are constituted by materials having different refractive indices.

13. An eye lens according to any of claims 1 to 12, characterized in that the near-range and far-range zones (N, F) are constituted by a material having radial refractive index gradients.

14. An eye lens according to any of claims 1 to 13, characterized in that the near-range and far-range zones (N, F) have been formed by a surface machining of the optical lens portion (1).

15. An eye lens according to claim 1, characterized in that the optically active surface is limited at the centre so as to provide a stenopaeic effect.

16. An eye lens according to claim 15, characterized in that a viewing hole is formed at the centre of the lens body and is bounded by lens material which is partly or entirely opaque.

**Revendications**

1. Lentille oculaire multifocale, notamment bifocale, intraoculaire artificielle comportant une partie de lentille (1) qui recouvre la pupille de l'iris et consiste en un matériel transparent, caractérisé en ce que la ou les zones de courte et de longue portée (N, F) occupent environ la même surface et sont disposées sur la partie de la lentille optique (1), ou de manière réciproquement concentrique, ou formées par une répartition diamétrale de la surface de la lentille en une zone de courte portée et une zone de longue portée, ou par la subdivision de la surface de la lentille en zones de courte portée et de longue portée s'étendant de manière radiale.

2. Lentille oculaire selon la revendication 1, caractérisée en ce que les zones de courte et de longue portées sont disposées de manière réciproquement concentriques.

3. Lentille oculaire selon la revendication 2, caractérisée en ce que les zones de courte et de longue portées (N, F) sont réparties en une surface circulaire intérieure et plusieurs surfaces annulaires circulaires concentriques disposées de manière alternante en direction radiale.

4. Lentille oculaire selon l'une des revendications 2 et 3, caractérisée en ce que la partie à portée longue (F) est disposée au centre de la partie de lentille optique (1).

5. Lentille oculaire selon la revendication 4, caractérisée en ce que l'effet optique des surfaces circulaires annulaires concentriques évolue progressivement de manière radiale vers l'extérieur.

6. Lentille oculaire selon l'une des revendications 2 et 3, caractérisée en ce que la partie courte portée (N) est disposée au centre de la partie de lentille optique et la partie longue portée (F) à l'extérieur.

7. Lentille oculaire selon la revendication 6, caractérisée en ce que l'effet optique des surfaces circulaires annulaires concentriques évolue progressivement de manière radiale vers l'intérieur.

8. Lentille oculaire selon la revendication 1, caractérisée en ce que les zones courte et longue portée (N, F) sont réparties en plusieurs secteurs !en direction radiale avec des angles identiques, qui sont disposés de manière alternante autour de l'axe optique.

9. Lentille oculaire selon la revendication 1, caractérisée en ce que les zones courte et longue portée (N, F) sont prévues chacune dans une moitié de la partie de lentille optique (1), et lorsque la lentille est implantée dans l'œil, la zone de transition entre la partie courte portée et la partie longue portée s'étend du bord supérieur de la lentille vers le bord inférieur et subdivise la partie de lentille optique (1) en une région de lentille nasale (plus près du nez du porteur) et une région de lentille temporale (éloignée du nez du porteur) et que la zone courte portée (N) se trouve dans la région nasale et la zone longue portée (F) dans la zone temporale de la lentille.

10. Lentille oculaire selon l'une des revendications 1 à 9, caractérisée en ce que les zones courte et longue portée (N, F) sont formées sur la surface avant et/ou la surface arrière de la partie de lentille optique (1).

11. Lentille oculaire selon la revendication 10, caractérisée en ce que la lentille est courbée de manière biconvexe pour obtenir l'effet longue portée et courte portée sur la surface avant ou arrière de la partie de lentille optique.

12. Lentille oculaire selon l'une des revendications 1 à 9, caractérisée en ce que les zones courte et longue portée (N, F) sont formées en matériaux à indices de réfraction différents.

13. Lentille oculaire selon l'une des revendications 1 à 12, caractérisée en ce que les zones courte et longue portée (N, F) sont formées par un matériau ayant des gradients d'indice de réfraction en direction radiale.

14. Lentille oculaire selon l'une des revendications 1 à 13, caractérisée en ce que les zones courte et longue portée (N, F) sont formées par l'usinage de surface de la partie de lentille optique.

15. Lentille oculaire selon la revendication 1, caractérisée en ce que la surface à action optique est limitée au centre de manière à produire un effet sténopéique.

16. Lentille oculaire selon la revendication 15, caractérisée en ce qu'au centre du corps de lentille est formé un trou de visée qui est bordé d'un matériau de lentille partiellement ou entièrement opaque.

# FIG. 1

FIG. 2

# FIG.3

# FIG.4